# EUROPEAN PATENT SPECIFICATION

(11) **EP 3 076 177 B1**
(45) Date of publication and mention of the grant of the patent: **01.08.2018**
(21) Application number: 14866585.4
(22) Date of filing: 01.12.2014
(51) Int. Cl.: G01N 33/543, G01N 33/531, G01N 33/576, G01N 33/558

(54) **IMMUNOCHROMATOGRAPHY-ASSISTED DETECTION METHOD**
IMMUNOCHROMATOGRAFIEUNTERSTÜTZTES DETEKTIONSVERFAHREN
PROCÉDÉ DE DÉTECTION ASSISTÉE PAR IMMUNOCHROMATOGRAPHIE

(30) Priority: 29.11.2013 JP 2013247279
(43) Date of publication of application: 05.10.2016
(73) Proprietor: Sekisui Medical Co., Ltd., Tokyo 103-0027 (JP)
(72) Inventor: NISHITANI, Kimiyoshi, Tokyo 103-0027 (JP)
(74) Representative: Hoffmann Eitle
(86) International application number: PCT/JP2014/081753
(87) International publication number: WO 2015/080286

(56) References cited:
- WO-A1-2005/007698
- WO-A1-2012/043746
- WO-A2-2007/092302
- JP-A- H10 332 699
- US-A1- 2012 034 711
- KOLOSOVA A YU ET AL: "Lateral-flow colloidal gold-based immunoassay for the rapid detection of deoxynivalenol with two indicator ranges", ANALYTICA CHIMICA ACTA, ELSEVIER, AMSTERDAM, NL, vol. 616, no. 2, 2 June 2008 (2008-06-02), pages 235-244, XP022659678, ISSN: 0003-2670, DOI: 10.1016/J.ACA.2008.04.029 [retrieved on 2008-04-20]
- DELMULLE BARBARA S ET AL: "Development of an immunoassay-based lateral flow dipstick for the rapid detection of aflatoxin B-1 in pig feed", JOURNAL OF AGRICULTURAL AND FOOD CHEMISTRY, AMERICAN CHEMICAL SOCIETY, US, vol. 53, no. 9, 1 May 2005 (2005-05-01), pages 3364-3368, XP002488362, ISSN: 0021-8561, DOI: 10.1021/JF0404804 [retrieved on 2005-04-08]
- GRITTI F ET AL: "Simultaneous measurement of pHWS and overloaded band profiles of small peptides when insufficiently buffered mobile phases are used in preparative liquid chromatography", JOURNAL OF CHROMATOGRAPHY A, ELSEVIER, AMSTERDAM, NL, vol. 1216, no. 51, 18 December 2009 (2009-12-18), pages 8874-8882, XP026783914, ISSN: 0021-9673, DOI: 10.1016/J.CHROMA.2009.10.026 [retrieved on 2009-11-04]
- MASATAKA ICHIKAWA ET AL.: 'Immunochromatography-ho to Koso Men'ekiho o Kumiawaseta Genri ni yoru Atarashii Influenza Jinsoku Shindan Kit (Espline Influenza A & B) no Kento' THE JOURNAL OF MEDICINE vol. 49, no. 3., March 2003, pages 469 - 478, XP002983639
- NAKAGAWA N ET AL.: 'Rapid detection and identification of two lineages of influenza B strains with monoclonal antibodies.' J VIROL METHODS. vol. 79, no. 1, April 1999, pages 113 - 120, XP027296682

## Description

### TECHNICAL FIELD

The present invention relates to an immunochromatographic detection method as defined in the appended claims. The present invention also relates to the method for detecting influenza B virus.

### BACKGROUND ART

A detection method using an immunochromatographic test strip is known as a method of detecting an analyte (a substance to be detected) in a sample through an antigen-antibody reaction. Immunochromatography is a method comprising spreading an immune complex formed by an analyte and a labeled conjugate (the labeled conjugate may also be referred to as a conjugate) in which an antibody to the analyte is immobilized, along with a mobile phase such as a buffer solution, through a stationary phase that is an insoluble membrane support having a detecting section on which an antibody to the analyte is immobilized as a capturing reagent, so as to detect the immune complex captured by the capturing reagent. Colloidal metal particles, such as colloidal gold, and colored latex particles are used as the label, and the degree of coloring of the detecting section can be used for determining the presence of and, in some cases, the amount of the analyte in the sample.

A typical configuration of the immunochromatographic test strip is a configuration that has a sample-supply portion (hereinafter also referred to as a sample pad) for supplying a sample, a conjugate pad for placing a conjugate, and a detecting section having a capturing reagent such as an antibody immobilized into a line shape, and that is disposed with an insoluble membrane support for capturing the aforementioned immune complex by allowing it to spread with mobile phase and an absorbent pad for absorbing, on the downstream side of the detecting section, the sample which has spread through the insoluble membrane support.

Methods of detecting a complex between an analyte and a conjugate in the detecting section of the immunochromatographic test strip having the aforementioned configuration include a method of measuring an intensity of reflected light from the label to calculate an absorbance (reflection absorption). The reflection absorption is calculated by using the ratio of the reflected light intensity measured at a detection line of an analyte in the detecting section and two neighboring parts thereof. When this method is used, however, a measurement waveform may be disturbed due to non-specific increases in the reflected light intensity in the vicinity of the upstream side and downstream side (particularly downstream side) of the detection line at which the immune complex is detected. When a sample containing an analyte at low concentration is used and the aforementioned complex is detected, a base line cannot be drawn due to the disturbance of the measurement waveform and, therefore, a specific signal (peak) cannot accurately be detected, which may make the measurement itself impossible. Since the frequency of occurrence of such disturbance of the measurement waveform and the degree or level of disturbance of the measurement waveform are not constant, the reproducibility of measurement is reduced and a correction to a suitable measurement value (in other words, validation of the measurement waveform) must be made in each measurement.

In a common immunochromatography using a white membrane, the disturbance of the measurement waveform can visually be observed as a phenomenon in which a corresponding part on the membrane looks relatively whiter than the color of the surroundings as if the part is bleached (a so-called white bleaching phenomenon; hereinafter also simply referred to as a white bleaching phenomenon or white bleaching).

Although the cause of such a white bleaching phenomenon is not clear, the phenomenon is often observed when an immunochromatographic test strip is used after being stored for a long period (e.g., one year or longer). The method of eliminating such a white bleaching phenomenon has not been known.

On the other hand, although immunochromatography has been put into practical use in a large number of rapid clinical tests (point-of-care-testing) and is particularly widely used in the diagnosis of infectious diseases such as influenza, immunochromatography has a problem of low detection sensitivity to influenza B virus as is the case with other immunoassays such as LTIA, and a method of eliminating this problem also has not been known.

US 2012/034711 relates to the field of biological detection and describes a multi-line immunochromatographic test strip for semi-quantitative detection of aflatoxin 1.

WO 2007/092302 describes a test device and related methods that provide for the detection of an analyte in a sample. The test devices provide for the rapid and accurate detection of a contamination in a sample from a biological fluid, environmental sample, or agricultural product.

JP H10 332699 relates to the field of immunochromatography and describes the analysis of compounds that can be extracted with an organic solvent from the test sample.

### CITATION LIST

### PATENT LITERATURE

Patent Document 1: WO 2012/043746

### SUMMARY OF INVENTION

### TECHNICAL PROBLEM

It is an object of the present invention to provide an immunochromatographic detection method capable of suppressing the occurrence of a so-called white bleaching phenomenon on a test strip for more accurate detection. It is also an object of the present invention to provide an immunochromatographic detection method enhancing the detection sensitivity particularly for influenza B virus.

### SOLUTION TO PROBLEM

As a result of intensive studies for solving the problems, surprisingly, the present inventors have found that in an immune reaction using an immunochromatographic test strip, the presence of methanol on the test strip suppresses the occurrence of the so-called white bleaching phenomenon of the test strip and enables favorable detection. More surprisingly, the present inventors found that the coexistence of methanol also enhances the detection sensitivity for influenza B virus, thereby completing the present invention. Therefore, the present invention has the following configuration.
[1] An immunochromatographic detection method using an immunochromatographic test strip comprising (1) a conjugate pad having a sample-supply portion for supplying a sample possibly containing an analyte, and a conjugate portion on the downstream side of the sample-supply portion, the conjugate portion containing a conjugate in which an antibody to the analyte is immobilized on a label, and (2) an insoluble membrane support having at least one detecting section on which an antibody to the analyte is immobilized,
   the method comprising the steps of:
   (A) supplying methanol and the sample possibly containing the analyte to the sample-supply portion; and
   (B) detecting the analyte as an immune reaction product on the insoluble membrane support, wherein the analyte is a virus or a protein.
[2] The detection method according to [1], wherein the step (A) is a step of supplying to the sample-supply portion a sample diluted in advance with a sample-diluting solution containing methanol.
[3] The detection method according to [1], wherein the step (A) is a step of diluting a sample with a sample-diluting solution containing methanol, and supplying the diluted sample to the sample-supply portion.
[4] The detection method according to any one of [1] to [3], wherein the analyte is influenza B virus, and wherein the antibody to the analyte immobilized on the label and the antibody to the analyte immobilized on the detecting section are anti-influenza B virus monoclonal antibodies.
[5] An immunochromatographic detection kit comprising:
   (a) an immunochromatographic test strip allowing a sample possibly containing an analyte to spread for detection of the analyte, the test strip comprising (1) a conjugate pad having a sample-supply portion for supplying the sample possibly containing the analyte, and a conjugate portion on the downstream side of the sample-supply portion,
      the conjugate portion containing a conjugate in which an antibody to the analyte is immobilized on a label, and (2) an insoluble membrane support having at least one detecting section on which an antibody to the analyte is immobilized; and
   (b) an immunochromatography sample-diluting solution containing methanol, wherein the analyte is a virus or a protein.
[6] The immunochromatographic detection kit according to [5], wherein the analyte is influenza B virus, and wherein the antibody to the analyte immobilized on the label and the antibody to the analyte immobilized on the detecting section are anti-influenza B virus monoclonal antibodies.
[7] The immunochromatographic detection kit according to [5] or [6], wherein (b) is an immunochromatography sample-diluting solution containing 0.1 to 20 % (v/v) methanol.
[8] The immunochromatographic detection kit according to any one of [5] to [7], wherein (b) is an immunochromatography sample-diluting solution containing 0.1 to 20 % (v/v) methanol in a buffer solution having pH of 6.0 to 10.0.
[9] Use of an immunochromatography sample-diluting solution for immunochromatographic influenza B virus-detection, the sample-diluting solution containing 0.1 to 20 % (v/v) methanol.
[10] Use of a sample-diluting solution according to [9], wherein the sample-diluting solution contains 0.1 to 20 % (v/v) methanol in a buffer solution having pH of 6.0 to 10.0.
[11] A use of an immunochromatography sample-diluting solution of (b) below in the manufacture of an influenza B virus detection kit comprising an immunochromatographic test strip of (a) below allowing a sample possibly containing influenza B virus to spread for detection of the virus:
   (a) an immunochromatographic test strip comprising (1) a conjugate pad having a sample-supply portion for supplying a sample possibly containing influenza B virus, and a conjugate portion on the downstream side of the sample-supply portion, the conjugate portion containing a conjugate in which an anti-influenza B virus antibody is immobilized on a label, and (2) an insoluble membrane support having at least one detecting section on which an anti-influenza B virus antibody is immobilized; and
   (b) an immunochromatography sample-diluting solution containing 0.1 to 20 % methanol.

In the present invention, immobilizing an antibody means that the antibody is physically or chemically carried by a label or an insoluble membrane support.

Detection in the present invention may be performed visually or by using an analyzing device and includes not only qualitative detection but also quantitative detection, i.e., measurement, for a quantifiable analyte.

In the present invention, upstream (upstream side) and downstream (downstream side) are defined such that the downstream (downstream side) indicates a direction of spreading of a mobile phase after a sample is supplied.

### ADVANTAGEOUS EFFECTS OF INVENTION

The present invention enables the suppression of occurrence of a so-called white bleaching phenomenon of an immunochromatographic test strip to achieve accurate measurement. In the detection of influenza virus, the present invention particularly enables sensitive detection of B virus associated with a problem of low detection sensitivity.

### BRIEF DESCRIPTION OF DRAWINGS

[Fig. 1] Fig. 1 is a schematic of an embodiment of an immunochromatographic test strip of the present invention.
[Fig. 2] Fig. 2 is a schematic of another embodiment of the immunochromatographic test strip of the present invention.
[Fig. 3] Fig. 3 is a schematic of a further embodiment of the immunochromatographic test strip of the present invention.

### DESCRIPTION OF EMBODIMENTS

### (Immunochromatographic Detection Method)

A detection method of the present invention is characterized by
using an immunochromatographic test strip comprising at least the following constituent elements (1) and (2):
(1) a conjugate pad having a sample-supply portion for supplying a sample possibly containing an analyte, and a conjugate portion on the downstream side of the sample-supply portion, the conjugate portion containing a conjugate in which an antibody to the analyte is immobilized on a label, and
(2) an insoluble membrane support having at least one detecting section on which an antibody to the analyte is immobilized, and
   comprising the following steps (A) and (B):
   (A) a step of supplying methanol and a sample possibly containing the analyte to the sample-supply portion, and
   (B) a step of detecting the analyte as an immune reaction product on the insoluble membrane support, wherein the analyte is a virus or a protein.

### (Addition of Methanol to Sample-Supply Portion)

The present invention is characterized by adding methanol to a sample-supply portion of a immunochromatographic test strip. The methanol can be added as 100 % methanol or as a methanol solution diluted with another solvent. Another solvent is preferably a water-soluble solvent and specific examples include purified water, saline, and low-concentration buffer solution having pH of 6.0 to 10.0. Since these water-soluble solvents are common with components of sample-diluting solution, if a diluted sample is used, methanol is preferably added to the sample-diluting solution and is added to the sample-supply portion together with the sample.

If an immunochromatographic test strip is designed as a dipstick type rather than a lateral-flow type, the test strip can be immersed in a solution containing methanol instead of adding methanol.

If methanol is diluted with a water-soluble solvent and added to a sample-supply portion, the methanol concentration in the water-soluble solvent is preferably 0.1 to 20.0 % (v/v), more preferably 0.5 to 18.0 % (v/v), 0.7 to 16.0 % (v/v), 1.0 to 15.0 % (v/v), 1.5 to 13.0 % (v/v), 1.8 to 12.0 % (v/v), 2.0 to 11.0 % (v/v), further more preferably 2.0 to 10 % (v/v). The concentration is also preferably 3.0 to 10.0 % (v/v), 4.0 to 10.0 % (v/v), or 5.0 to 10.0 % (v/v) in some cases.

Since methanol is volatile, methanol can be added to a solvent at the time of use, or a methanol containing solvent adjusted to the concentration in advance and stored in a highly airtight container can be used.

### (Sample-Diluting Solution)

Since a sample-diluting solution is used for diluting a sample in accordance with the concentration of an analyte in the sample, a diluting solution of any composition may be used unless the diluting solution significantly inhibits an antigen-antibody reaction or, conversely, significantly accelerates the reaction, resulting in excessive aggregation of labels and thereby causing poor results in the spreading by capillarity or making it impossible to detect the signal of antigen-antibody reaction corresponding to the concentration of the antigen.

For example, the sample-diluting solution is desirably purified water, saline, or low-concentration buffer solution having pH of 6.0 to 10.0. The buffer solution more desirably has pH of 6.5 to 9.5, 7.0 to 9.0, and 7.5 to 8.5.

Examples of the buffer solution include, for example, a 10 to 20 mmol/L phosphate buffer solution, a 10 to 20 mmol/L Tris-HCl buffer solution, and a 10 to 20 mmol/L Bis-Tris buffer solution. A surfactant can be added to these diluting solutions for the purpose of controlling a spread rate of the sample on the strip.

### (Addition of Sample)

In the present invention, a sample is supplied to the sample-supply portion directly without dilution or after being diluted with the sample-diluting solution and filtrated as need.

Examples of the sample possibly containing an analyte include a substance mainly derived from a living body (organism), such as biological fluid, and extraction liquid acquired by extracting an analyte therefrom. Specific examples of the substance derived from a living body (organism) include blood, urine, feces, nasal secretions and nasal secretion aspirates derived from the nostril, the nasal cavity, the pharynx, and the nasopharynx, secretions collected as a sputum or a swab specimen, and saliva. In particular, if the analyte is an influenza virus, the sample is preferably a nasal secretion and a nasal secretion aspirate derived from the nostril, the nasal cavity, the pharynx, and the nasopharynx, a secretion collected as a sputum or a swab specimen, etc.

### (Analyte)

In the present invention, the analyte include viruses and physiologically active substances such as proteins that may generally be measured by utilizing an antigen-antibody reaction, as defined in the appended claims.

The viruses include, for example, influenza viruses such as influenza A virus and influenza B virus, hepatitis B virus, hepatitis C virus, and human immunodeficiency virus, and the proteins include, for example, human hemoglobin, a hepatitis B virus antibody, a hepatitis C virus antibody, and a human immunodeficiency virus antibody. In particular, preferably, an influenza virus is used as an analyte and, more preferably, a plurality of detecting sections described later are formed to use influenza A virus and influenza B virus as analytes. Although the reason is not clear, particularly, the present invention enables the detection of influenza B virus with higher sensitivity as compared to the conventional techniques.

### (Detection)

A method of detecting an analyte as an immune reaction product on the insoluble membrane support may be any method capable of detecting a signal derived from the label and may be a known method. For example, if the label is colloidal gold, the absorbance or the reflected light intensity may be detected and, if the label is colored latex, the coloring intensity may be detected.

According to the present invention, since the occurrence of the so-called white bleaching phenomenon is suppressed even in the case of optical detection using a white insoluble membrane, a waveform without disturbance is acquired so that the detection and the measurement can accurately be performed.

### (Antibody to Analyte)

In the present invention, an antibody to an analyte is an antibody capable of immunologically specifically binding to the analyte. Antibodies to an analyte are immobilized on a label and a detecting section described later. Although the antibodies immobilized on the label and the detecting section may be the same, preferably, different antibodies are used for the label and the detecting section. By differentiating the antibody or antigen immobilized on the label and the antibody immobilized on the detecting section, an acquired immunochromatographic test strip can suppress competition between a reaction of an analyte bound to a conjugate with the antibody or antigen of the detecting section and a reaction of the analyte bound to the conjugate with an unreacted conjugate, and can increase the reactivity between the analyte bound to the conjugate and the antibody of the detecting section, resulting in favorable sensitivity of the immunochromatographic test strip. The different antibodies mean that types are different from each other, and specifically refer to antibodies recognizing different epitopes.

Preferably, the antibodies immobilized on the label and the detecting section are monoclonal antibodies. By using monoclonal antibodies, the specificity of reaction can be increased.

If an analyte is an influenza virus, antibodies immobilized on the label and the detecting section may be any antibodies capable of detecting the influenza virus and, preferably, the antibodies are anti-influenza virus monoclonal antibodies such as an anti-influenza A virus monoclonal antibody and an anti-influenza B virus monoclonal antibody.

In addition to whole molecules of these antibodies, functional fragments of antibodies having antigen-antibody reaction activity are also considered as antibodies in the present invention. Functional fragments of antibodies include those acquired through immunization of an animal as well as those acquired by using a generecombination technique, and chimeric antibodies. Functional fragments of antibodies include, for example, F(ab')₂ and Fab'. These functional fragments can be produced by treating the antibodies with proteolytic enzymes (e.g., pepsine and papain). By using F(ab')₂ that is a functional fragmented antibody as the label, for example, a size of a conjugate having an antibody bound to the label can be reduced, resulting in an excellent spreading property in the conjugate pad and the insoluble membrane support. For some analytes, the specificity of reaction can be increased by using a functional fragmented antibody.

### (Label)

A label for labeling these antibodies is preferably colloidal gold particles, platinum colloid particles, colored latex particles, and magnetic particles and is particularly preferably colored latex particles.

Colored latex particles can be prepared by preparing polystyrene-based particles through soap-free polymerization without using an emulsifier in accordance with the method described in paragraph [0022] of Japanese Laid-Open Patent Publication No. H06-306108, for example, and then in accordance with the method described in paragraphs [0025] to [0035] of the same document, and colored particles commercially available from Seradyn, Inc. and Magsphere Inc. are also usable. In the following description, the case of using colored latex particles as the label will be described in detail.

### (Conjugate)

The conjugate used in the present invention is the label as described above having an antibody immunologically reactive with an analyte immobilized thereon. If the analyte is an influenza virus, preferably, the conjugate is a colored latex particle having an anti-influenza virus monoclonal antibody immobilized thereon.

The antibody is typically immobilized on the colored latex particle by a chemical bond and, in such a case, an antibody concentration is preferably adjusted to 1 mg/mL to 5 mg/mL and a buffer solution and pH are preferably a 20 mmol/L MES buffer solution (pH 5.5-6.5) or a 50 mmol/L borate buffer solution (pH 8-9), more preferably a 20 mmol/L MES buffer solution (pH 6.5). A region not bound to an antibody on the colored latex particle is preferably bound to BSA or the like for blocking. Colored latex particle-labeled antibodies produced in this way are dispersed and stored in a storage reagent for inhibiting denaturation. For this denaturation inhibitor, protein such as BSA, glycerol, sugar, and the like are used.

### (Sample Pad)

In the present invention, a "sample pad" is a part playing a role of a sample-supply portion receiving a sample and is disposed upstream of a conjugate pad described later in contact with the former pad. The sample pad may be of any material and form absorbing a liquid sample and allowing the passage of the liquid and the component of an analyte.

Specific examples of materials suitable for sample pad include, but not limited to, glass fibers, acrylic fibers, hydrophilic polyethylene material, dry paper, paper pulp, fabric, and the like. A glass fiber pad is preferably used.

Although the sample pad is disposed upstream of the conjugate pad and can be in contact with the conjugate pad, the sample pad can additionally be given a function of the conjugate pad. In other words, the sample pad is also the conjugate pad and does not have to be separated. In this case, the same pad has the function of a sample pad on the upstream side and the function of a conjugate pad on the downstream side and this configuration will be described in paragraphs related to the conjugate pad.

The sample pad may contain a commonly used blocking reagent, a buffer solution component, a blood coagulating agent or the like if a sample is blood as needed provided that the object of the present invention is achieved and the reaction system is not affected. In this case, the blocking reagent, the buffer solution component, the blood coagulating agent or the like may be contained in at least a portion of the sample pad or may be contained in the whole of the sample pad.

### (Conjugate Pad)

The conjugate pad used in the present invention is made of a pad-shaped porous material capable of allowing a sample to spread after passing through the sample pad and capable of retaining the conjugate, and retains the conjugate in a portion or the whole thereof.

The conjugate pad contains a conjugate and, if contained in a portion, the conjugate is preferably retained in a line shape orthogonal to the direction of spreading of the sample, for example.

A line width of the line-shaped conjugate portion may be such a width that the amount of conjugate necessary for detecting the analyte can be contained, and is desirably 3 to 5 mm.

The conjugate pad is stacked on an insoluble membrane support such that the lower surface of the downstream end portion thereof is in contact with the upper surface of the insoluble membrane support described later. The conjugate pad is stacked on the insoluble membrane support such that the lower surface of its sample-supply portion is not in contact with the upper surface of the insoluble membrane support while the lower surface of its conjugate portion is in contact with the upper surface of the insoluble membrane support. The stacking is achieved if the lower surface of the conjugate pad comes into contact with the upper surface of the insoluble membrane support, and it is not necessary to fix the surfaces. A contact portion between the lower surface of the conjugate pad and the upper surface of the insoluble membrane support may be a portion of the lower surface of the conjugate pad and is desirably a half or more of the lower surface. It is also desirable that the entire lower surface of the conjugate pad is in contact with the upper surface of the insoluble membrane support. In a conjugate pad, an analyte (influenza virus) in the sample and a conjugate (colored latex particle with the anti-influenza virus monoclonal antibody immobilized thereon) form a complex (aggregate). The sample is then allowed to spread into the insoluble membrane support disposed in contact with the lower surface of the conjugate portion.

If a same pad has the function of a sample pad and the function of a conjugate pad as described above, a conjugate portion is formed in a downstream portion of the pad while a sample-supply portion is formed in an upstream portion, and this sample-supply portion is a portion playing the role of a sample pad. When a sample possibly containing an analyte is supplied to the sample-supply portion of the conjugate pad, the sample flows from the sample-supply portion on the upstream side through a porous material portion without the conjugate to the conjugate portion on the downstream side.

With regard to the porous material making up the conjugate pad, the pad may be made of paper, nonwoven fibers of a cellulose mixture, nitro cellulose, polyester, acrylonitrile copolymer, glass, rayon, or the like. In particular, a pad made of glass fibers (glass fiber pad) is preferable.

### (Insoluble membrane support)

The insoluble membrane support used in the present invention has at least one detecting section on which an antibody immunologically reactive with an analyte is immobilized. The antibody immunologically reactive with an analyte can be immobilized on the insoluble membrane support by a conventionally known method. In the case of a lateral flow immunochromatographic test strip, the immobilization is performed as follows. After a solution containing a predetermined concentration of the antibody is prepared, the solution is applied in a line shape to the insoluble membrane support by using an apparatus having a mechanism capable of moving a nozzle in a horizontal direction while discharging the solution at a constant rate from the nozzle, and is dried for immobilization.

The concentration of the antibody in the solution is preferably 0.1 to 5 mg/mL, more preferably 0.5 to 2 mg/mL. The amount of the antibody immobilized on the insoluble membrane support can be optimized by adjusting the discharge rate from the nozzle of the apparatus in the case of the lateral flow type and the preferred rate is 0.5 to 2 µL/cm.

The measurement method using the lateral flow immunochromatographic test strip is a measurement method in which the sample supplied from a portion of the conjugate pad spreads such that the sample moves in a direction parallel to the insoluble membrane support due to the capillary phenomenon, the portion of the conjugate pad being in contact with the insoluble support.

The solution containing a predetermined concentration of the antibody can be prepared by adding an antibody to a buffer solution. A type of the buffer solution may be a commonly used buffer solution such as a phosphate buffer solution, a Tris buffer solution, and a Good's buffer solution. The buffer solution preferably has pH in a range of 6.0 to 9.5, more preferably 6.5 to 8.5. The buffer solution may further contain salts such as sodium chloride, a stabilizer such as sucrose and a preservative, and antiseptic such as Proclin. The salts includes those contained for adjusting ion strength, such as sodium chloride, as well as those added for the purpose of adjusting pH of the buffer solution, such as sodium hydroxide.

After the antibody is immobilized on the insoluble membrane support, the insoluble membrane support can be coated and blocked with a commonly used blocking agent in a form of solution or vapor, except the part on which the antibody is immobilized.

A control capture reagent conventionally used for an immunochromatographic test strip may be immobilized on the insoluble membrane support. The control capture reagent is a reagent for ensuring the reliability of assay and captures a control reagent contained in the conjugate pad. For example, if labeled KLH is contained as a control reagent in the conjugate pad, an anti-KLH antibody and the like corresponds to the control capture reagent. A position of immobilization of the control capture reagent can appropriately be selected in accordance with the design of the assay system.

A membrane making up the insoluble membrane support used in the present invention can be a known membrane conventionally used as an insoluble membrane support of an immunochromatographic test strip. For example, the membrane may be made of fibers of polyethylene, polyethylene terephthalate, nylons, glass, polysaccharide such as cellulose and cellulose derivatives, ceramics, and the like. Specifically, the membrane may be glass fiber filter paper, cellulose filter paper, or the like, commercially available from Sartorius, Millipore, Toyo Roshi, Whatman, etc. In particular, UniSart CN140 from Sartorius is preferable. By selecting the pore diameter and the structure of the insoluble membrane support as needed, the rate of flow of the complex between the conjugate and the analyte in the sample can be controlled in the insoluble membrane support.

### (Absorbent Pad)

In the immunochromatographic test strip of the present invention, preferably, an absorbent pad is disposed on the downstream end portion of the insoluble membrane support. The absorbent pad is a part having liquid absorbability controlling the spread of the sample by absorption of the sample moving in and passing through the insoluble membrane support. The absorbent pad can be a known absorbent pad conventionally used for an immunochromatographic test strip and, for example, filter paper can be used. Preferably, Whatman 740-E is used.

### (Immunochromatographic Test Strip)

The immunochromatographic test strip of the present invention includes at least a conjugate pad and an insoluble membrane support. The conjugate pad and the insoluble membrane support are stacked such that the lower surface of the conjugate pad is in contact with the upper surface of the insoluble membrane support. The conjugate pad is disposed such that the lower surface of the conjugate portion of the conjugate pad is partially or entirely in contact with the upper surface of the insoluble membrane support. As described above, preferably, an absorbent pad is disposed on the downstream end portion of the insoluble membrane support.

The immunochromatographic test strip is preferably disposed on a solid phase backing such as a plastic adhesive sheet. The solid phase backing is made of a material not hindering the capillary flow of the sample and the conjugate. The immunochromatographic test strip may be fixed to the solid phase backing with an adhesive or the like. In this case, an adhesive component or the like are also made of a material not hindering the capillary flow of the sample and the conjugate. A polyester film or the like can be used for lamination in order to increase the mechanical strength of the insoluble membrane support and to prevent evaporation of moisture (drying) during the assay. The immunochromatographic test strip can be used after stored in or mounted on an appropriate container (housing) with consideration given to the size of the immunochromatographic test strip, the method and the position of addition of the sample, the position of formation of the detecting section of the insoluble membrane support, the signal detection method, etc., and such a stored/mounted state is referred to as a "device."

The immunochromatographic test strip of the present invention comprises a conjugate pad and an insoluble membrane support and may include another reagent or constituent element depending on the measurement condition and the sample. Another reagent may be a blocking agent preventing non-specific reactions, for example, and another constituent element may be a 3rd pad for removing a component in the sample unnecessary for measurement.

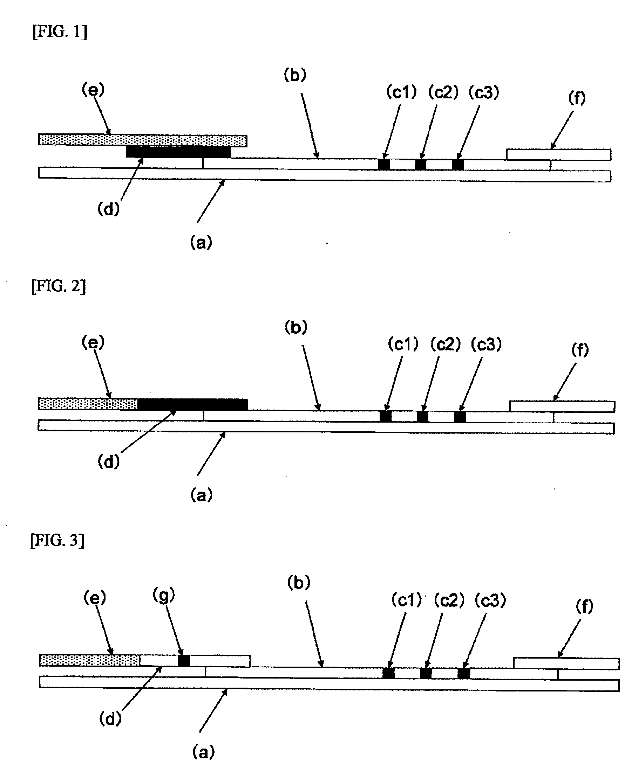

Fig. 1 shows a schematic of typical structure of the immunochromatographic test strip of the present invention.

After the antibody-immobilized membrane (b) is affixed to the plastic adhesive sheet (a), the conjugate pad (d) is disposed and mounted, the sample pad (e) is also disposed and mounted to overlap the conjugate pad, and the absorbent pad (f) is disposed and mounted on the other end. The antibody-immobilized membrane (b) has an anti-influenza A virus monoclonal antibody (c1), an anti-influenza B virus monoclonal antibody (c2), and a control antibody (c3) immobilized in a line shape such that an A-line, a B-line, and a control line, respectively, appear when the sample passes through.

An example of the immunochromatographic test strips having such a structure is Rapid Testa (registered trademark) FLUII (manufactured by Sekisui Medical Co. Ltd.).

Fig. 2 shows a structure in which the conjugate pad (d) plays the role of the sample pad (e) in the test strip having the structure of Fig. 1. Fig. 3 shows a structure in which the conjugate portion (g) is formed in a small portion of the sample pad.

An example of the immunochromatographic test strip having such a structure is Rapid Testa (registered trademark) color FLU stick (manufactured by Sekisui Medical Co. Ltd.).

### (Kit)

The immunochromatographic detection kit of the present invention comprises at least the immunochromatographic test strip and the immunochromatography sample-diluting solution containing methanol, wherein the analyte is a virus or a protein as claimed.

Although the present invention has been described in terms of a sandwich type detection for an analyte that is an antigen, the present invention is also applicable to the case that an analyte is an antibody and a competitive type detection, and a lectin, a receptor, a nucleic acid chain are also usable instead of an antigen as long as specific binding to an analyte can be made.

### EXAMPLES

Although the present invention will specifically be described with examples, the scope of the present invention is not limited to these examples.

### Example 1 (White Bleaching Evaluation Test)

White bleaching was evaluated by using immunochromatographic test strips (manufactured by Sekisui Medical Co. Ltd., Rapid Testa (registered trademark) Color FLU Stick, Lot.K1221221077P) stored unopened for two years at the temperature described in the package insert. Glycerol or methanol, respectively, was added to the accompanying specimen-diluting solution (Tris buffer solution (pH 8.5)) of the test strips to prepare specimen-diluting solutions containing 1 %, 5 %, and 10 % glycerol or specimen-diluting solutions containing 1 %, 5 %, and 10 % methanol based on the whole volume. A group (n=3) of the immunochromatographic test strips were immersed in each of the specimen-diluting solutions to allow the specimen-diluting solutions to spread on the chromatograph for 10 minutes. Subsequently, the occurrence of white bleaching on each of the test strips was visually checked. The result is shown in Table 1.

Similarly, the test strips were immersed in specimen-diluting solutions containing 1 %, 5 %, and 10 % ethanol to conduct the same test The result is shown in Table 2.

"A line" and "B line" in the tables indicate parts on which the anti-influenza A virus monoclonal antibody and the anti-influenza B virus monoclonal antibody, respectively, were immobilized, and are portions forming a colored line when each of the viruses is detected. "Cont." indicates a part on which the control capturing reagent, i.e., anti-KLH antibody, was immobilized, and the part forms a colored line when KLH in the conjugate is detected. The evaluation results in the tables are evaluated based on criteria including "-" indicative of "no white bleaching", "+" indicative of "very thin and slight white bleaching", "++" indicative of "thin line of white bleaching at a level having no effect on determination", "+++" indicative of "somewhat thick line of white bleaching at a level having no effect on determination", and "++++" indicative of "thick line of white bleaching at a level having an effect on determination". These notations are common to the subsequent tests.

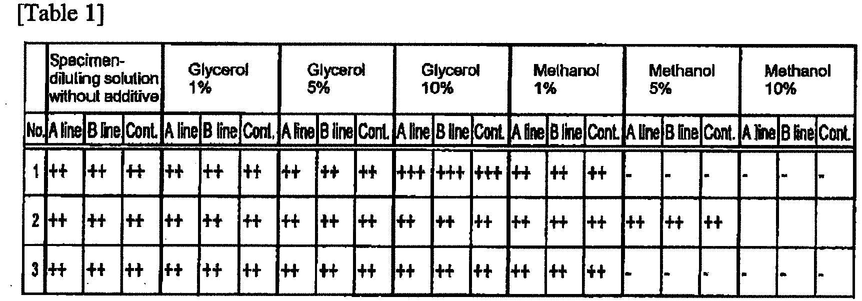

**[Table 2]**

| | Ethanol 1% | | | Ethanol 5% | | | Ethanol 10% | | |
|---|---|---|---|---|---|---|---|---|---|
| No. | A line | B line | Cont. | A line | B line | Cont | A line | B line | Cont. |
| 1 | +++ | +++ | +++ | +++ | +++ | +++ | +++ | +++ | +++ |
| 2 | ++++ | ++++ | ++++ | +++ | +++ | +++ | +++ | +++ | +++ |
| 3 | +++ | +++ | +++ | +++ | +++ | +++ | +++ | +++ | +++ |

### (Results)

When the performance of the specimen-diluting solution having none of glycerol, methanol, and ethanol added thereto is considered as a control, the white bleaching occurred at the same level when the glycerol-added specimen-diluting solutions were used, while the occurrence of the white bleaching was significantly suppressed when the specimen-diluting solution containing 5 % or 10 % methanol was used.

On the other hand, when the ethanol-containing specimen-diluting solutions were used, the occurrence of the white bleaching was not suppressed and the degree of the white bleaching increased instead.

The coloring intensity of "Cont." obtained based on a color sample was 2.5 to 3.75 in all the cases and was within a normally usable range.

### Example 2 (Acceleration Test)

The immunochromatographic test strips immediately after production and the immunochromatographic test strips stored at 60 °C for 10 days and 20 days were immersed in the specimen-diluting solutions containing 5 % and 10 % methanol to allow the solutions to spread for 10 minutes as was the case with Example 1 and the occurrence of white bleaching was visually checked. The result is shown in Table 3.

**[Table 3]**

| | Specimen-diluting solution without additive | | | | | | Methanol 5 % added | | | | | | Methanol 10 % added | | | | | |
|---|---|---|---|---|---|---|---|---|---|---|---|---|---|---|---|---|---|---|
| | 0time | | 60°C 10d | | 60°C 20d | | 0time | | 60°C 10d | | 60°C 20d | | 0time | | 60°C 10d | | 60°C 20d | |
| No | A line | B line | A line | B line | A line | B line | A line | B line | A line | B line | A line | B line | A line | B line | A line | B line | A line | B line |
| 1 | ++ | ++ | - | - | - | - | + | + | - | - | + | + | - | - | - | - | + | + |
| 2 | ++ | ++ | + | + | + | + | + | + | + | + | + | + | - | - | - | - | - | - |
| 3 | +++ | +++ | + | + | + | + | - | - | - | - | + | + | - | - | - | - | - | - |
| 4 | + | + | + | + | ++ | ++ | - | - | + | + | - | - | - | - | - | - | - | - |
| 5 | ++ | ++ | ++ | ++ | + | + | + | + | - | - | + | + | - | - | - | - | - | - |

| | | | | | | | | | | | | | | | | | | |
|---|---|---|---|---|---|---|---|---|---|---|---|---|---|---|---|---|---|---|
| d: days | | | | | | | | | | | | | | | | | | |

### (Results)

When the specimen-diluting solutions containing 5 % and 10 % methanol were used, suppression of the occurrence of and reduction in the degree of white bleaching were recognized in all the immunochromatographic test strips immediately after production and stored at 60 °C for 10 days and 20 days. Particularly when the specimen-diluting solution containing 10 % methanol was used, a significant suppression of the occurrence of white bleaching was recognized.

### Example 3(Comparison of Coloring Intensity in A-line and in B-line)

The immunochromatographic test strips were used for the comparison of the coloring intensity in "A line" as well as in "B line." Samples were prepared by mixing 330 µL of the specimen-diluting solutions containing 5 % and 10 % methanol with 50 µL of specimens acquired by diluting inactivated virus antigens of influenza A and influenza B virus strains at the dilution fold described in Tables 4 and 5 (described below the respective types of influenza viruses in tables). A group (n=2) of the test strips were immersed in 135 µL of the samples to which the respective inactivated virus antigens were added, to allow the specimen-diluting solutions to spread on the test strips for 10 minutes. Subsequently, the coloring intensity at a detecting section of each of the viruses is obtained based on a color sample. The results of influenza A virus and influenza B virus are shown in Table 4 and Table 5, respectively.

**[Table 4]**

| **A H1N1** | | | | |
|---|---|---|---|---|
| | | Conv. | Meth. 5% | Meth. 10% |
| | No. | A line | A line | A line |
| A/H1 pdm lot.111026 1/20 | 1 | 1.75 | 2 | 1.75 |
| | 2 | 2 | 2 | 2 |
| A/Brisbane/59/2007 lot.090608 1/100 | 1 | 1.5 | 1.5 | 1.5 |
| | 2 | 1.5 | 1.25 | 1.5 |
| A/Solomon Islands/3/2006 lot.070615 1/20 | 1 | 2.25 | 2.5 | 2.25 |
| | 2 | 2.25 | 2.25 | 2.25 |

| **A H3N2** | | | | |
|---|---|---|---|---|
| | | Conv. | Meth. 5% | Meth. 10% |
| | No. | A line | A line | A line |
| A/Kitakyushu/159/93 lot.110614 1/400 | 1 | 1.5 | 1.5 | 1.5 |
| | 2 | 1.5 | 1.5 | 1.5 |
| A/Uruguay/716/2007 lot.090608 1/100 | 1 | 1.25 | 1.5 | 1.25 |
| | 2 | 1.5 | 1.25 | 1.25 |
| A/Wisconsin/67/2005 lot.070615 1/200 | 1 | 1 | 1.25 | 1.25 |
| | 2 | 1.25 | 1.25 | 1.25 |

| | | | | |
|---|---|---|---|---|
| Conv.: Conventional Meth.: Methanol | | | | |

**[Table 5]**

| | | Conv. | Meth. 5% | Meth. 10% |
|---|---|---|---|---|
| | n= | B line | B line | B line |
| B/Lee/40 lot.110610 1/100 | 1 | 1.5 | 1.75 | 2 |
| | 2 | 1.75 | 2 | 2 |
| B/Florida/4/2006 lot.100521 1/50 | 1 | 1.5 | 1.75 | 2 |
| | 2 | 1.5 | 1.75 | 1.75 |
| B/Shanghai/361/2002 lot.050813-1 1/50 | 1 | 2.25 | 2.75 | 2.75 |
| | 2 | 2.25 | 2.75 | 2.75 |

| | | | | |
|---|---|---|---|---|
| Conv.: Conventional Meth.: Methanol | | | | |

### (Results)

When the inactivated antigen of influenza A virus was used, a change in the coloring intensity was recognized in none of the strains regardless of addition of 5 % and 10 % methanol to the specimen-diluting solutions. On the other hand, when the inactivated antigen of influenza B virus was used, an increase in the coloring intensity was recognized due to the addition of methanol.

### INDUSTRIAL APPLICABILITY

The immunochromatographic detection method of the present invention can provide an accurate detection method without a so-called white bleaching of a test strip. Particularly when applied to the detection of influenza B virus, the present invention can provide a detection method excellent in sensitivity as compared to the conventional methods.

### REFERENCE SIGNS LIST

(a) Plastic adhesive sheet
(b) Antibody-immobilized membrane
(c1) Anti-influenza A virus monoclonal antibody
(c2) Anti-influenza B virus monoclonal antibody
(c3) Control antibody
(d) Conjugate pad
(e) Sample pad
(f) Absorbent pad
(g) Conjugate portion

## Claims

1. An immunochromatographic detection method using an immunochromatographic test strip comprising (1) a conjugate pad having a sample-supply portion for supplying a sample possibly containing an analyte, and a conjugate portion on the downstream side of the sample-supply portion, the conjugate portion containing a conjugate in which an antibody to the analyte is immobilized on a label, and (2) an insoluble membrane support having at least one detecting section on which an antibody to the analyte is immobilized,
the method comprising the steps of:
(A) supplying methanol and the sample possibly containing the analyte to the sample-supply portion; and
(B) detecting the analyte as an immune reaction product on the insoluble membrane support,
wherein the analyte is a virus or a protein.

2. The detection method according to claim 1, wherein said virus is selected from the group consisting of influenza A virus, influenza B virus, hepatitis B virus, hepatitis C virus, and human immunodeficiency virus and said protein is selected from the group consisting of human hemoglobin, a hepatitis B virus antibody, a hepatitis C virus antibody, and a human immunodeficiency virus antibody.

3. The detection method according to claim 1 or 2, wherein the step (A) is a step of supplying to the sample-supply portion a sample diluted in advance with a sample-diluting solution containing methanol.

4. The detection method according to claim 1 or 2, wherein the step (A) is a step of diluting a sample with a sample-diluting solution containing methanol, and supplying the diluted sample to the sample-supply portion.

5. The detection method according to any one of claims 1 to 4, wherein the analyte is influenza B virus, and wherein the antibody to the analyte immobilized on the label and the antibody to the analyte immobilized on the detecting section are anti-influenza B virus monoclonal antibodies.

6. An immunochromatographic detection kit comprising:
(a) an immunochromatographic test strip allowing a sample possibly containing an analyte to spread for detection of the analyte, the test strip comprising (1) a conjugate pad having a sample-supply portion for supplying the sample possibly containing the analyte, and a conjugate portion on the downstream side of the sample-supply portion, the conjugate portion containing a conjugate in which an antibody to the analyte is immobilized on a label, and (2) an insoluble membrane support having at least one detecting section on which an antibody to the analyte is immobilized; and
(b) an immunochromatography sample-diluting solution containing methanol, wherein the analyte is a virus or a protein.

7. The immunochromatographic detection kit according to claim 6, wherein the analyte is influenza B virus, and wherein the antibody to the analyte immobilized on the label and the antibody to the analyte immobilized on the detecting section are anti-influenza B virus monoclonal antibodies.

8. The immunochromatographic detection kit according to claim 6 or 7, wherein (b) is an immunochromatography sample-diluting solution containing 0.1 to 20 % (v/v) methanol.

9. The immunochromatographic detection kit according to any one of claims 6 to 8, wherein (b) is an immunochromatography sample-diluting solution containing 0.1 to 20 % (v/v) methanol in a buffer solution having pH of 6.0 to 10.0.

10. Use of an immunochromatography sample-diluting solution for immunochromatographic influenza B virus-detection, the sample-diluting solution containing 0.1 to 20 % (v/v) methanol.

11. Use of a sample-diluting solution according to claim 10, wherein the sample-diluting solution contains 0.1 to 20 % (v/v) methanol in a buffer solution having pH of 6.0 to 10.0.

12. A use of an immunochromatography sample-diluting solution of (b) below in the manufacture of an influenza B virus detection kit comprising an immunochromatographic test strip of (a) below allowing a sample possibly containing influenza B virus to spread for detection of the virus:
(a) an immunochromatographic test strip comprising (1) a conjugate pad having a sample-supply portion for supplying a sample possibly containing influenza B virus, and a conjugate portion on the downstream side of the sample-supply portion, the conjugate portion containing a conjugate in which an anti-influenza B virus antibody is immobilized on a label, and (2) an insoluble membrane support having at least one detecting section on which an anti-influenza B virus antibody is immobilized; and
(b) an immunochromatography sample-diluting solution containing 0.1 to 20 % methanol.

## Patentansprüche

1. Immunchromatographisches Detektionsverfahren unter Verwendung eines immunchromatographischen Teststreifens, umfassend (1) eine Konjugatkontaktstelle mit einem Probenbereitstellungsteil zum Bereitstellen einer Probe, welche möglicherweise einen Analyten enthält, und einem Konjugatteil am hinteren Ende des Probenbereitstellungsteils, wobei der Konjugatteil ein Konjugat enthält, in welchem ein Antikörper gegen den Analyten an einer Markierung immobilisiert ist, und (2) einen unlöslichen Membranträger mit wenigstens einem Detektionsabschnitt, auf welchem ein Antikörper gegen den Analyten immobilisiert ist,
wobei das Verfahren die Schritte umfasst:
(A) Bereitstellen von Methanol und der Probe, welche möglicherweise den Analyten enthält, im Probenbereitstellungsteil; und
(B) Detektieren des Analyten als ein Immunreaktionsprodukt auf dem unlöslichen Membranträger, wobei der Analyt ein Virus oder ein Protein ist.

2. Detektionsverfahren gemäß Anspruch 1, wobei das Virus ausgewählt ist aus der Gruppe, bestehend aus Influenza A Virus, Influenza B Virus, Hepatitis B Virus, Hepatitis C Virus und humanem Immundefizienzvirus und das Protein ausgewählt ist aus der Gruppe, bestehend aus humanem Hämoglobin, einem Hepatitis B Virus-Antikörper, einem Hepatitis C Virus-Antikörper und einem humanen Immundefizienzvirus-Antikörper.

3. Detektionsverfahren gemäß Anspruch 1 oder 2, wobei der Schritt (A) ein Schritt des Bereitstellens einer Probe, welche im Voraus mit einer Methanol enthaltenden Probenverdünnungslösung verdünnt wurde, im Probenbereitstellungsteil ist.

4. Detektionsverfahren gemäß Anspruch 1 oder 2, wobei der Schritt (A) ein Schritt des Verdünnens einer Probe mit einer Methanol enthaltenden Probenverdünnungslösung und des Bereitstellens der verdünnten Probe im Probenbereitstellungsteil ist.

5. Detektionsverfahren gemäß einem der Ansprüche 1 bis 4, wobei der Analyt Influenza B Virus ist, und wobei der an einer Markierung immobilisierte Antikörper gegen den Analyten und der auf dem Detektionsabschnitt immobilisierte Antikörper gegen den Analyten monoklonale anti-Influenza B Virus-Antikörper sind.

6. Immunchromatographisches Detektionskit, umfassend:
(a) einen immunchromatographischen Teststreifen, welcher einer Probe, welche möglicherweise einen Analyten enthält, erlaubt sich für die Detektion des Analyten auszubreiten, wobei der Teststreifen umfasst: (1) eine Konjugatkontaktstelle mit einem Probenbereitstellungsteil zum Bereitstellen der Probe, welche möglicherweise den Analyten enthält, und einem Konjugatteil am hinteren Ende des Probenbereitstellungsteils, wobei der Konjugatteil ein Konjugat enthält, in welchem ein Antikörper gegen den Analyten an einer Markierung immobilisiert ist, und (2) einen unlöslichen Membranträger mit wenigstens einem Detektionsabschnitt, auf welchem ein Antikörper gegen den Analyten immobilisiert ist; und
(b) eine Methanol enthaltende Immunchromatographie-Probenverdünnungslösung,
wobei der Analyt ein Virus oder ein Protein ist.

7. Immunchromatographisches Detektionskit gemäß Anspruch 6, wobei der Analyt Influenza B Virus ist, und wobei der an einer Markierung immobilisierte Antikörper gegen den Analyten und der auf dem Detektionsabschnitt immobilisierte Antikörper gegen den Analyten monoklonale anti-Influenza B Virus-Antikörper sind.

8. Immunchromatographisches Detektionskit gemäß Anspruch 6 oder 7, wobei (b) eine Immunchromatographie-Probenverdünnungslösung ist, welche 0,1 bis 20% (v/v) Methanol enthält.

9. Immunchromatographisches Detektionskit gemäß einem der Ansprüche 6 bis 8, wobei (b) eine Immunchromatographie-Probenverdünnungslösung ist, welche 0,1 bis 20% (v/v) Methanol in einer Pufferlösung mit einem pH-Wert von 6,0 bis 10,0 enthält.

10. Verwendung einer Immunchromatographie-Probenverdünnungslösung zur immunchromatographischen Detektion von Influenza B Virus, wobei die Probenverdünnungslösung 0,1 bis 20% (v/v) Methanol enthält.

11. Verwendung einer Probenverdünnungslösung gemäß Anspruch 10, wobei die Probenverdünnungslösung 0,1 bis 20% (v/v) Methanol in einer Pufferlösung mit einem pH-Wert von 6,0 bis 10,0 enthält.

12. Verwendung einer Immunchromatographie-Probenverdünnungslösung gemäß untenstehend (b) bei der Herstellung eines Detektionskits für Influenza B Virus, umfassend einen immunchromatographischen Teststreifen gemäß untenstehend (a), welcher einer Probe, welche möglicherweise Influenza B Virus enthält, erlaubt sich für die Detektion des Virus auszubreiten:
(a) ein immunchromatographischer Teststreifen, umfassend (1) eine Konjugatkontaktstelle mit einem Probenbereitstellungsteil zum Bereitstellen einer Probe, welche möglicherweise Influenza B Virus enthält, und einem Konjugatteil am hinteren Ende des Probenbereitstellungsteils, wobei der Konjugatteil ein Konjugat enthält, in welchem ein anti-Influenza B Virus-Antikörper an einer Markierung immobilisiert ist, und (2) einen unlöslichen Membranträger mit wenigstens einem Detektionsabschnitt, auf welchem ein anti-Influenza B Virus-Antikörper immobilisiert ist; und
(b) eine Immunchromatographie-Probenverdünnungslösung, welche 0,1 bis 20% (v/v) Methanol enthält.

## Revendications

1. Procédé de détection immunochromatographique utilisant une bandelette de test immunochromatographique comprenant (1) un tampon de conjugué comportant une partie de fourniture d'échantillon pour fournir un échantillon contenant éventuellement un analyte, et une partie de conjugué sur le côté aval de la partie de fourniture d'échantillon, la partie de conjugué contenant un conjugué dans lequel un anticorps dirigé contre l'analyte est immobilisé sur un marqueur, et (2) un support de membrane insoluble comportant au moins une section de détection sur laquelle un anticorps dirigé contre l'analyte est immobilisé,
le procédé comprenant les étapes de :
(A) fourniture de méthanol et de l'échantillon contenant éventuellement l'analyte à la partie de fourniture d'échantillon ; et
(B) détection de l'analyte sous forme de produit de réaction immunitaire sur le support de membrane insoluble,
dans lequel l'analyte est un virus ou une protéine.

2. Procédé de détection selon la revendication 1, dans lequel ledit virus est choisi dans le groupe constitué du virus de la grippe A, du virus de la grippe B, du virus de l'hépatite B, du virus de l'hépatite C, et du virus de l'immunodéficience humaine et ladite protéine est choisie dans le groupe constitué de l'hémoglobine humaine, d'un anticorps du virus de l'hépatite B, d'un anticorps du virus de l'hépatite C, et d'un anticorps du virus de l'immunodéficience humaine.

3. Procédé de détection selon la revendication 1 ou 2, dans lequel l'étape (A) est une étape de fourniture à la partie de fourniture d'échantillon d'un échantillon dilué à l'avance avec une solution de dilution d'échantillon contenant du méthanol.

4. Procédé de détection selon la revendication 1 ou 2, dans lequel l'étape (A) est une étape de dilution d'un échantillon avec une solution de dilution d'échantillon contenant du méthanol, et de fourniture de l'échantillon dilué à la partie de fourniture d'échantillon.

5. Procédé de détection selon l'une quelconque des revendications 1 à 4, dans lequel l'analyte est le virus de la grippe B, et dans lequel l'anticorps dirigé contre l'analyte immobilisé sur le marqueur et l'anticorps dirigé contre l'analyte immobilisé sur la section de détection sont des anticorps monoclonaux anti-virus de la grippe B.

6. Kit de détection immunochromatographique comprenant :
(a) une bandelette de test immunochromatographique permettant à un échantillon contenant éventuellement un analyte de diffuser pour la détection de l'analyte, la bandelette de test comprenant (1) un tampon de conjugué comportant une partie de fourniture d'échantillon pour fournir l'échantillon contenant éventuellement l'analyte, et une partie de conjugué sur le côté aval de la partie de fourniture d'échantillon, la partie de conjugué contenant un conjugué dans lequel un anticorps dirigé contre l'analyte est immobilisé sur un marqueur, et (2) un support de membrane insoluble comportant au moins une section de détection sur laquelle un anticorps dirigé contre l'analyte est immobilisé ; et
(b) une solution de dilution d'échantillon pour immunochromatographie contenant du méthanol, dans lequel l'analyte est un virus ou une protéine.

7. Kit de détection immunochromatographique selon la revendication 6, dans lequel l'analyte est le virus de la grippe B, et dans lequel l'anticorps dirigé contre l'analyte immobilisé sur le marqueur et l'anticorps dirigé contre l'analyte immobilisé sur la section de détection sont des anticorps monoclonaux anti-virus de la grippe B.

8. Kit de détection immunochromatographique selon la revendication 6 ou 7, dans lequel (b) est une solution de dilution d'échantillon pour immunochromatographie contenant 0,1 à 20 % (v/v) de méthanol.

9. Kit de détection immunochromatographique selon l'une quelconque des revendications 6 à 8, dans lequel (b) est une solution de dilution d'échantillon pour immunochromatographie contenant 0,1 à 20 % (v/v) dans une solution tampon ayant un pH de 6,0 à 10,0.

10. Utilisation d'une solution de dilution d'échantillon pour immunochromatographie pour la détection immunochromatographique du virus de la grippe B, la solution de dilution d'échantillon contenant 0,1 à 20 % (v/v) de méthanol.

11. Utilisation d'une solution de dilution d'échantillon selon la revendication 10, dans laquelle la solution de dilution d'échantillon contient 0,1 à 20 % (v/v) de méthanol dans une solution tampon ayant un pH de 6,0 à 10,0.

12. Utilisation d'une solution de dilution d'échantillon pour immunochromatographie de (b) ci-dessous dans la fabrication d'un kit de détection du virus de la grippe B comprenant une bandelette de test immunochromatographique de (a) ci-dessous permettant à un échantillon contenant éventuellement le virus de la grippe B de diffuser pour la détection du virus :
(a) une bandelette de test immunochromatographique comprenant (1) un tampon de conjugué comportant une partie de fourniture d'échantillon pour fournir un échantillon contenant éventuellement le virus de la grippe B, et une partie de conjugué sur le côté aval de la partie de fourniture d'échantillon, la partie de conjugué contenant un conjugué dans lequel un anticorps anti-virus de la grippe B est immobilisé sur un marqueur, et (2) un support de membrane insoluble comportant au moins une section de détection sur laquelle un anticorps anti-virus de la grippe B est immobilisé ; et
(b) une solution de dilution d'échantillon pour immunochromatographie contenant 0,1 à 20 % de méthanol.
